# EUROPEAN PATENT APPLICATION

(11) **EP 4 674 429 A1**
(43) Date of publication of application: **07.01.2026**
(21) Application number: 24186124.4
(22) Date of filing: 02.07.2024
(51) Int. Cl.: A61K 38/48, A61P 7/10, A61P 29/00, A61K 36/746, A61K 36/63, A61K 36/49, A61K 36/48

(54) **COMPOSIZIONE PER IL TRATTAMENTO DELL'EDEMA E DEL LINFEDEMA**

(71) Applicant: Anvest Group S.r.l., 20152 Milano (IT)
(72) Inventor: ALFIERI, Dario, 84086 Roccapiemonte (IT); ROBERTO, Dario, 84086 Roccapiemonte (IT); SANDRO, Dario, 84083 Castel S. Giorgio (IT)
(74) Representative: Cattaneo, Elisabetta

(57) **Abstract**

The invention relates to a composition comprising extracts of natural ingredients for treating edema and/or lymphedema.

## Description

### FIELD OF THE INVENTION

The present invention relates to a composition comprising extracts of natural ingredients for treating edema and/or lymphedema.

### BACKGROUND ART

Chronic inflammatory diseases represent the most significant cause of death in the world. The World Health Organization (WHO) ranks chronic diseases as the greatest threat to human health. The prevalence of diseases associated with chronic inflammation is projected to steadily increase over the next 30 years in the United States. In 2000, nearly 125 million Americans were living with chronic conditions, and 61 million (21%) had more than one. According to estimates by the Rand Corporation, in 2014 nearly 60% of Americans had at least one chronic condition, 42% had more than one, and 12% of adults had 5 or more chronic conditions. Worldwide, 3 out of 5 people die from chronic inflammatory diseases such as stroke, chronic respiratory disease, heart disease, cancer, obesity and diabetes.

Inflammation, per se, represents a vital defense mechanism for health, triggered by a variety of factors, including pathogens, damaged cells, and toxic compounds. The inflammation acts, thus removing the harmful stimuli and initiating the healing process. Usually, during acute inflammatory responses, cellular and molecular events and interactions effectively minimize impending injuries or infections. Therefore, such a mitigation process contributes to the restoration of tissue homeostasis and to resolution of acute inflammation.

However, uncontrolled acute inflammation can become chronic, potentially leading to tissue and organ damage, and contributing to the onset of a variety of chronic diseases, including cardiovascular and intestinal diseases, lymphedema, diabetes, arthritis, and cancer.

At the tissue level, inflammation is characterized by the 5 key signs of the inflammation process which are: heat (*calor*)*,* redness (*rubor*)*,* swelling (*tumor*)*,* pain (*dolor*) and functional alteration (*functio laesa*) which can be explained by increased blood flow in the area of interest, high cellular metabolism, vasodilation, release of soluble mediators, cellular influx and extravasation of liquids (edema), plasma proteins and leukocytes (changes attributable to the actions of local microcirculation).

Acute and chronic inflammation is also associated with changes in microvascular form and function. Microcirculation is the terminal vascular network of the systemic circulation consisting of micro-vessels with a diameter less than 20µm, in turn formed by arterioles, post-capillary venules, capillaries and the subcellular constituents thereof. Microcirculation is the final destination of the cardiovascular system and the main surface for the exchange of gases, fluids and nutrients between blood and tissues. Arterioles, which are the most proximal segments of the microvascular system, are internally lined by a monolayer of endothelial cells, connected to each other by tight and adherent junctions, the main role of which is to control the pressure, flow and supply of nutrients to the capillary beds.

At rest, endothelial cells maintain a non-thrombogenic, non-adhesive and therefore non-reactive surface at the interface between blood and tissue. This means that in the basal state thereof, endothelial cells form a sufficient barrier for retaining most proteins, including albumin. The oncotic effect of these plasma proteins serves to limit the extravasation of liquids. Furthermore, resting endothelial cells cannot recruit leukocytes and this is largely attributed to the absence of luminal molecules capable of capturing these cells from circulation so as to allow a limited immune surveillance. Similarly, the chemokines that activate the leukocytes are also absent.

However, some leukocyte adhesion molecules (e.g., P-selectin and some chemokines such as IL-8) are expressed, but are sequestered inside secretory vesicles (WPB) and thus unavailable to circulating leukocytes. Basal NO production in endothelial cells can also potentially exert anti-inflammatory effects by inhibiting leukocyte activation. Following activation by pro-inflammatory mediators, the endothelium undergoes changes, becoming an important participant in the generation and evolution of phlogosis.

Therefore, microcirculation is highly reactive and vitally participates in the inflammatory response. All the segments of microcirculation (arterioles, capillaries, and venules) show characteristic phenotypic changes during inflammation which appear to be directed at improving the release of inflammatory cells to damaged/infected tissue, isolating the region from healthy tissue and systemic circulation, and laying the foundation for tissue repair and regeneration. The best characterized responses of microcirculation to inflammation comprise compromised vasomotor function, reduced capillary perfusion, leukocyte and platelet adhesion, activation of the coagulation cascade and increased thrombosis, increased vascular permeability, and an increased rate of blood and lymphatic system proliferation.

Edema, on the other hand, is a swelling due to the expansion of the volume of interstitial liquid in tissues or in an organ which can occur in numerous forms, including unilateral, bilateral, localized or generalized edema. Several clinical conditions present with edema, making it a critical clinical feature for diagnostic medicine.

The formation of edema occurs in two fundamental phases.

First, an alteration of capillary hemodynamics which favors the movement of liquids from the vascular space to the interstitial space. Initially, the fluid moves from the vascular space to the interstitial space and consequently reduces plasma volume and tissue perfusion. To respond to these changes, the kidney retains sodium and water. This compensation mechanism targets the return of liquids into the vascular space due to the concentration difference. However, this change in capillary hemodynamics leads to the retention of fluids entering the interstitial space and causing edema.

Edema can also form in response to high capillary hydraulic pressures or increased capillary permeability (which occurs during phlogosis), destruction of the endothelial glycocalyx, decreased interstitial compliance, lower plasma oncotic pressure, or a combination of these factors.

In particular, the increase in capillary permeability, typically due to vascular damage, causes edema for several reasons. When the vessels are damaged, the porosity of the capillary walls increases and, consequently, the net filtration increases. Furthermore, the protein coefficient through the capillary wall decreases, thereby reducing the difference between the oncotic pressure of the capillary and the oncotic pressure below the endothelial glycocalyx. The oncotic pressure gradient is reduced, thus edema results.

Furthermore, lymphatic obstruction is a well-known cause of edema and the most common causes include lymphedema, tumors, fibrosis, inflammation, infections, surgeries, and congenital abnormalities.

In fact, our body maintains health and prevents diseases by virtue of the coordinated action of different organs and apparatuses, among which the lymphatic system is of primary importance. The lymphatic system consists of three main components:
- lymph, i.e., interstitial fluid, originating from the extravasation of liquid and proteins from blood capillaries;
- lymphatic vessels, which drain lymph from throughout the body into the bloodstream;
- lymphocytes, cells of the immune system which concentrate in the lymph nodes and control lymphatic content, also for the presence of microbes and viruses.

Lymphatic vessels originate in the peripheral tissues and convey lymph into venous circulation. These vessels also transport antigens and immune cells to draining lymph nodes, which promote immune response or tolerance. In addition to participating in immunity, the lymphatic system is involved in many other physiological processes, of which circulation and metabolism are the most important. The lymphatic system collects the lymph that is filtered by the arterial side of the capillary bed and also transports dietary fats, hormones and waste substances.

Lymphatic vessels transport lymph from the periphery to venous circulation. Similar to blood vessels, they can have different sizes, from small lymphatic capillaries (or initial lymphatic vessels) to large collecting vessels (lymphatic ducts or collecting lymphatic vessels). Lymphatic vessels have one-way valves and mainly consist of lymphatic endothelial cells (LECs) and lymphatic muscle cells (LMCs). The dysfunction of any of these components can lead to several diseases, the most common of which is lymphedema.

The accumulation of liquids, already determined by the presence of phlogosis, further stimulates the activation of inflammatory cells, which in turn modify the extracellular matrix, decreasing lymphatic function. Several inflammatory cells participate in the etiopathogenesis thereof, mainly T lymphocytes and macrophages.

Firstly, macrophages are distinguished into two populations (M1 and M2) based on the transcriptional profile thereof and the cytokines produced, which determine the effects thereof in favoring or inhibiting the phlogistic process.
- M1 macrophages (pro-inflammatory) intervene in the first phase of inflammation and eliminate threats and invaders, by virtue of the production of reactive oxygen and nitrogen intermediates and pro-inflammatory cytokines (IL-1b, TNF-α, IL-6).
- M2 macrophages (anti-inflammatory) have an anti-inflammatory function, a high phagocytic capacity and are mainly involved in wound healing and tissue repair. In particular, they block the action of M1 macrophages, "cleaning" the area by eliminating the remaining debris and producing important physiological factors, capable of repairing and rebuilding the damaged tissue so as to promote a correct revascularization (lymphangiogenesis).

Not all the mechanisms which include the balance of macrophage phenotypes M1 and M2 as well as the possible polarization thereof influenced by the pathophysiological condition are currently clarified.

Leukotrienes are synthesized in leukocytes starting from arachidonic acid (AA) by the action of several enzymes, especially including 5-lipoxygenase (LOX-5). This step leads to the formation of the conjugated triene epoxide LTA4 converted by the enzyme LTA4 hydrolase (LTA4H) into LTB4, recently identified as one of the key molecules in the pathogenesis of lymphedema, as well as one of the most pro-inflammatory molecules present in the body.

LTB4 exerts the biological activity thereof after binding to the receptors coupled to related G proteins, called LTB4R and LTB4R2, with which signal transduction is initiated.

Thereby, LTB4 is capable of controlling CD8+ and CD4+ cells and consequently recruiting neutrophils and pro-inflammatory macrophages in lymphedematous tissues (ability to recruit and activate immune cells). Such cells, in particular neutrophils, produce more LTB4 upon activation, consequently recruiting even more leukocytes into the inflammatory site, triggering a veritable vicious phlogistic cycle.

The excessive recruitment and activation of neutrophils by LTB4 can cause tissue damage, thus contributing to the pathological features and progression of lymphedema. Furthermore, LTB4 stimulates Th17 differentiation and acts as a molecular link between adaptive and innate immunity in lymphedema. LTB4 also modulates VEGFR-3 and Notch signaling, both important in lymphangiogenesis. In this regard, high concentrations of LTB4 counteract lymphangiogenesis, consequently reducing the ability to drain extravasated liquids. Low concentrations of LTB4 instead promote the formation of new lymphatic pathways and the restoration of damaged ones.

Therefore, lymphedema is a chronic inflammatory disease characterized by an accumulation of protein-rich fluid which occurs when the ability of the lymphatic system to transport interstitial liquid is exceeded. Therefore, lymphedema represents a pathological state characterized by insufficient lymphatic drainage related to obstructions or functional alterations of the lymphatic vessels; consequently, such obstruction manifests an increase in interstitial fluids and edema forms. These types of edemas are characterized by a high protein content exudate, which can therefore fibrotize, making it more difficult for macrophages to lyse, which are adapted to mobilize edematous accumulation.

In the United States and Western countries, lymphedema most commonly occurs as a complication of lymph node dissection for cancer treatment. It is estimated that 30-50% of patients who undergo lymph node dissection will develop lymphedema. Lymphedema can also occur after less invasive procedures such as sentinel node dissection, thus putting almost all cancer survivors at risk of this dreaded complication. Although lymphedema most commonly occurs as a complication of breast cancer management, it is also frequently seen in patients treated for other solid malignant tumors. In fact, a recent meta-analysis of nearly 8000 patients reported an overall incidence of 16% in patients treated for gynecological neoplasms, melanoma, urological, sarcoma, and malignant tumors of the head and neck.

Patients with lymphedema have chronic and progressive swelling, pain, recurrent infections, and a significant decrease in quality of life. The swelling can progress significantly, causing serious consequences.

Lymphedema is classified as primary when it results from genetic mutations, is rare and is usually caused by malformations of the lymphatic vessels and/or a malfunction of lymphatic drainage. More than 20 genes have been associated with various forms of primary lymphedema, but the mutations thereof can explain only a portion of all cases of primary lymphedema, and a high degree of genetic heterogeneity is found among patients with lymphedema. The disease can occur at birth (congenital) or evolve during childhood, puberty or even adulthood, and can be diagnosed through genetic screening methods.

Secondary lymphedema results from lymphatic damage caused by different agents, such as infection, surgery, or trauma. Secondary lymphedema typically manifests following therapeutic interventions related to the neoplasm. In fact, surgical procedures usually lead to the dissection or excision of lymph nodes. Radiation therapy can also damage the dermal lymphatic vessels and cause lymph node fibrosis. The combination of radiation and surgery increases the risk of lymphedema by four to eight times. Lastly, even the obstruction of lymphatic vessels by adipose tissue and reduced physical activity, typical of pathological obesity, can cause secondary lymphedema. Numerous diseases of orthopedic relevance, especially those of surgical interest, can be complicated by acute lymphedema from prolonged immobilization. The various diseases in question include bone fractures, muscle tears, acute capsulo-ligamentous diseases, ligament tears, contusions, tibio-tarsal sprains and traumas, including surgical ones. The moment of surgery also directly interferes with the coagulation cascade process, resulting in a postoperative thrombophilic state characterized by several elements, such as the activation of platelets and the coagulation cascade, increase in fibrinogen and factor VIII, compromised fibrinolytic activity, stasis and alteration of blood flow (from prolonged immobilization), direct damage to the vascular endothelium. Certain diseases such as urinary tract calculosis, testicular lymphedema, hydrocele, as well as surgical techniques (transurethral resection of the prostate, radical prostatectomy, penis degloving after fracture, phimosis, testicular detorsion) also involve the formation of edema and the onset of pain caused by the extravasation of intravasal liquids.

Diseases related to inflammation and edema are difficult to place within a specific category, since they are linked to very variable causes and very different symptoms. Current knowledge in the field of phlogistics and the most common therapeutic strategies follow the cause-effect concept. The different molecules participating in the inflammatory cascade are known, whereby the most used drugs, for example acetylsalicylic acid, naproxen, ibuprofen, diclofenac, nimesulide, ketoprofen, carry out very specific mechanisms of action aimed at limiting the development thereof and the related consequences.

The limit in drug therapy, for example in the case of NSAIDs (Non-Steroidal Anti-Inflammatory Drugs) lies in the fact that the operation thereof is limited to a single mechanism of action which acts at a specific level of the complex cascade of phlogosis. Furthermore, NSAIDs are known for the gastrolesive or hepatotoxic side effects thereof in the event of prolonged therapies. The gastrolesive effect of NSAIDs is linked to the action thereof of inhibiting the enzyme COX1 (Cyclooxygenase 1) responsible for the production of prostaglandin E2, which at the gastric level leads to the normal formation of mucus which protects the inner walls of the stomach from the acidity of HCl (hydrochloric acid). The blockage of COX2 (Cyclooxygenase 2) limits mucosal vascularization and platelet aggregation.

Few therapeutic strategies are currently available for the resolution of symptoms closely related to edema and the extravasation of liquids from blood and lymphatic vessels.

First-line intervention in the event of edema and lymphedema is represented by the management of inflammation, the first triggering cause of extravasation, as well as the partial reduction of vessel permeability in order to limit the progress of the pathological condition.

Therefore, the need to find a new multimodal therapeutic approach which is not limited to resolving symptoms (acute anti-inflammatory therapy), but which simultaneously considers the different biochemical actors which cause venous and lymphatic extravasation, and ensures the maintenance of the delicate physiological balances thereof, is very much felt.

Therefore, it is the object of the present invention to treat edema and lymphedema effectively, overcoming the adverse effects of the prior art.

### SUMMARY OF THE INVENTION

The inventors of the present invention have surprisingly found a combination of active ingredients which was able to effectively treat edema and/or lymphedema by virtue of a synergistic activity between the active ingredients, as will be evident from the experimental section.

The inventors have found that the specific combination of active ingredients is particularly and preferably suitable for use in treating lymphedema.

Therefore, the inventors have identified a combination of active ingredients which was found to have a greater synergistic effect than the additive combination of the known ingredients, as will be apparent from the experimental section.

The same combined active ingredients produce a synergistic effect which allows both edema and, preferably, lymphedema to be treated effectively and surprisingly. Therefore, the invention relates to a composition comprising a *Morinda Citrifolia* extract, a *Meliloto Officinalis* extract, a *Quercus Robur* extract, an olive extract and bromelain.

Advantageously, the active ingredient bromelain is in microencapsulated form. The composition of the invention can be used for the formulation of a nutraceutical product, being it a supplement or a food, by using suitable excipients.

The invention relates to a nutraceutical product, preferably a supplement, comprising the composition of the invention and physiologically acceptable excipients.

The composition of the invention, i.e., the nutraceutical product, the food or the supplement comprising the same, has demonstrated an excellent activity in reducing inflammation markers, in regulating vascular permeability, in the ability to reabsorb extravasation, in the maintenance of lymph node function, in the ability to restore cellular oncotic pressure and in the stimulation of lymphangiogenesis.

Therefore, in another aspect, the invention relates to the composition or nutraceutical (or supplement or food) of the invention for use in treating lymphedema.

According to a further aspect, the invention relates to the composition or nutraceutical product (or supplement or food) of the invention for use in treating edema.

In an advantageous aspect of the invention, the composition of the invention can be combined with at least one known drug, preferably in treating edema and/or lymphedema.

In a further aspect, the invention relates to the use of the nutraceutical product in improving symptoms associated with edema or lymphedema, in which said improvement consists of reducing inflammation markers, regulating vascular permeability, the ability to reabsorb extravasation, the maintenance of lymph node function, the ability to restore cellular oncotic pressure and the stimulation of lymphangiogenesis.

Therefore, in another aspect, the invention relates to a combination comprising
- the composition according to the invention or the nutraceutical according to the invention and
- a drug
for simultaneous or separate use in treating edema and/or lymphedema

In fact, the composition of the invention, i.e., the nutraceutical product, supplement or food, can be advantageously employed as a co-therapeutic agent in a combination therapy with a known drug.

Advantageously said known drug is selected from the group consisting of a non-steroidal anti-inflammatory drug (NSAID), preferably selected from the group consisting of acetylsalicylic acid, ibuprofen, naproxen, ketoprofen and diclofenac, a diuretic drug, preferably furosemide, and an anti-edema drug, preferably selected from the group consisting of heparan sulfate, troxerutin and diosmin, more preferably it is an NSAID, even more preferably it is ketoprofen.

Advantageous aspects of the composition and the supplement of the invention, such as quantities, posology and dosages will be indicated in detail in the detailed description and the surprising synergistic effects deriving therefrom will be evident from the subsequent experimental section.

### DESCRIPTION OF THE DRAWINGS

Figure 1 shows the leg thickness intensity at different times after the administration of the composition of the invention comprising a *Morinda Citrifolia* extract, a *Meliloto Officinalis* extract, a *Quercus Robur* extract, an olive extract and bromelain, in rats with paw edema induced by carrageenan injection. Each point represents the mean ± standard deviation of 6 mice. t0: 50.0%; t1: 40.0%; t2: 35.0%; t3: 32.0%; t4: 31.0%; t5: 30.5%; t6: 30.3%. * P <0.01 and ** P <0.001, compared to the healthy leg.
Figure 2 shows the effect of the composition of the invention on the proportion of F4/80 and CD11b+ cells in the RAW264.7 macrophages (* P <0.01 and ** P <0.001) of Example 2.7.
Figure 3 shows the effect of the composition of the invention on TNF-α expression in the RAW264.7 macrophages of Example 4. * P <0.01 and ** P <0.001.
Figure 4 shows the effect of the composition of the invention on IL-6 expression in the RAW264.7 macrophages of Example 4. * P <0.01 and ** P <0.001.
Figure 5 shows the effect of the composition of the invention on IL-1β expression in the RAW264.7 macrophages of Example 4. * P <0.01 and ** P <0.001.
Figure 6 shows the Western Blot analysis of the expression of the surface marker CD206 related to the M2 macrophages of Example 3. * P <0.01 and ** P <0.001.
Figure 7 shows the Western Blot analysis of the expression of the surface marker Arg-1 related to the M2 macrophages of Example 3. * P <0.01 and ** P <0.001.
Figure 8 shows the Western Blot analysis of IL-10 expression related to the M2 macrophages of Example 3. * P <0.01 and ** P <0.001.
Figure 9 shows the LTB4 levels in the plasma processed according to the ELISA method according to Example 5.1.
Figure 10 shows the plasma protein levels according to Example 5.2.

### DETAILED DESCRIPTION OF THE INVENTION

The inventors have surprisingly identified a combination of active ingredients which was found to have a greater synergistic effect than the additive combination of the known ingredients, as will be apparent from the experimental section.

The same combined active ingredients produce a synergistic effect which allows both edema and, preferably, lymphedema to be treated effectively and surprisingly. Therefore, the invention relates to a composition comprising a *Morinda Citrifolia* extract, a *Meliloto Officinalis* extract, a *Quercus Robur* extract, an olive extract and bromelain.

In the present invention, the following is used:
- "edema" means a swelling due to the expansion of the volume of interstitial liquid in tissues or in an organ;
- "lymphedema" means an edema affecting the lymphatic system;
- "physiologically acceptable solvent" means a solvent that does not produce significant adverse reactions when introduced into the human body or applied to the human body;

- "nutraceutical product" means a nutraceutical composition with appropriate excipients added, also defined as a supplement or food; and
- "preparation unit dose" means the total dose of the reference pharmaceutical form to express the quantities of the active ingredients or substances contained in the pharmaceutical form itself, for example a capsule.

The composition of the invention comprises bromelain.

Bromelain is a proteolytic enzyme obtained from the juice and stem of pineapples. Advantageously, the bromelain according to the invention is microencapsulated. Even more advantageously, the microencapsulated bromelain according to the invention has a proteolytic enzymatic activity equal to about 5000 GDU/g.

The composition of the invention, by virtue of the presence of bromelain, is advantageously capable of hydrolyzing the peptide bond of inflammatory protein molecules with consequent inactivation of the latter.

The composition of the invention comprises bromelain in standard form. In an advantageous and preferred form of the invention, the bromelain of the composition is microencapsulated.

Without being bound to any theory, the inventors have thus advantageously allowed the bromelain contained in the composition to be protected from the acidity of the gastric environment, thus improving the breakability of the peptide ligands of the composition itself.

In a more advantageous and preferred form, the inventors of the present invention used microencapsulated bromelain having a proteolytic enzymatic activity equal to about 5000 GDU/g.

The composition is thus more stable and the bioavailability for therapeutic purposes thereof is maximized.

The liquids forming the edema are held in place by protein structure mediators (cytokines, interleukins, TNF-α and fibrin). The composition of the invention, by virtue of the bromelain, is capable of hydrolyzing and inactivating these protein structures, allowing the return of vascular liquids to the vascular bed and therefore the reabsorption of the edema and the reduction of pro-inflammatory mediators which feed phlogosis. By virtue of the presence of bromelain, the composition of the invention,
- also stimulates diuresis, allowing the removal of considerable volumes of liquids through the excretion of urine;
- acts on the arachidonic acid pathway: it modulates pro-inflammatory prostaglandins (by means of the inhibition of prostaglandin E2 and thromboxane A2)

- enhances anti-inflammatory mediators, increasing platelet cyclic adenosine monophosphate (cAMP) and, therefore, prostaglandin (PG) I2 and PGE1 levels;
- modulates immunity through cell migration, acting on the migration of neutrophils towards sites of inflammation (diapedesis).

In detail, by virtue of the presence of bromelain, the composition removes several molecules from the cell surface (including CD128a/CXCR1, CD128b/CXCR2, CD14, CD44, CD16 and CD21) that are vital for leukocyte traffic, cell adhesion and the induction of pro-inflammatory mediators, allowing the composition to be effective in various conditions associated with edema and lymphedema.

Therefore, the composition of the invention has no gastrointestinal side effects, contrary to the NSAIDs generally used for treating edema and lymphedema.

Preferably the bromelain, more preferably in microencapsulated form, still more preferably having a proteolytic enzymatic activity equal to about 5000 GDU/g, is present in the composition of the invention in the range from about 24% to about 94%, preferably from about 40% to about 59%, more preferably about 47% w/w with respect to the total weight of the composition.

The composition of the invention comprises a *Morinda Citrifolia* extract.

*Morinda Citrifolia* extract is an extract obtained from the plant belonging to the Rubiaceae family, preferably it is a dry extract obtained from the fruits of the plant. By virtue of the *Morinda Citrifolia* extract, the composition of the invention showed anti-inflammatory effects. TNF-α is known to be a pro-inflammatory cytokine which contributes to extensive tissue damage and plays a key role in the upregulation of other pro-inflammatory cytokines and the induction of iNOS by macrophages. The inhibition of the inflammatory response generated by the composition of the invention manages phlogosis and edema. Macrophages are important players in the phlogistic process, as they present antigen to lymphocytes, exert phagocytosis, and produce cytokines and active substances which regulate immune functions, including TNF-α, IL-1β, and nitric oxide (NO). The modulation of these functions by the macrophages activated by the composition of the invention helps treating the inflammation which generates edema.

Advantageously and preferably, the *Morinda Citrifolia* extract according to the present invention is titrated in rubiadin, preferably at 1% in rubiadin, measured by HPLC chromatography.

The *Morinda Citrifolia* extract is preferably present in the composition of the invention in the range from about 6% to about 24% w/w, more preferably about 12% w/w.

The composition of the invention comprising the *Morinda Citrifolia* extract titrated in rubiadin was even more effective in the treatment of edema and lymphedema.

The composition of the invention comprises a *Meliloto Officinalis* extract.

The *Meliloto Officinalis* extract is preferably present in the composition of the invention in the range from about 9% to about 35% w/w with respect to the total weight of the composition, more preferably about 18% w/w.

*Meliloto Officinalis* extract is an extract obtained from the plant belonging to the Fabaceae (or Leguminous) family, preferably it is a dry extract obtained from the flowering tops of the plant.

The main constituents of *Meliloto Officinalis* are coumarin glycosides (melylotoside, umbelliferone, scopoletin), flavonoids (kaempferol, quercetin), and phenolic acids (caffeic acid and derivatives). Then there are saponins (melilotosaponin, soya saponin, astragoloside VIII) and tannins. By virtue of the *Meliloto Officinalis* extract, the composition of the invention was effective against high-protein edema, as in the case of lymphedema. Advantageously, by virtue of the presence of *Meliloto Officinalis* extract, the composition of the invention showed anti-inflammatory effects, favoring both the polarization of macrophages towards the anti-inflammatory phenotype M2, and the inhibition of the pro-inflammatory phenotype M1, responsible for the synthesis of inflammation mediators. Furthermore, the composition of the invention comprising a *Meliloto Officinalis* extract allowed a control/restoration of the oncotic pressure underlying the lymphedema by modulating the physiological permeability of the lymphatic vessels.

Advantageously and in a preferred form of the invention, the *Meliloto Officinalis* extract according to the present invention is titrated in coumarin, preferably at 40% in coumarin by HPLC chromatography.

The composition of the invention comprises a *Quercus Robur* extract.

Preferably, the *Quercus Robur* extract is present in the composition of the invention in the range from about 6% to about 24% w/w with respect to the total weight of the composition, more preferably about 12% w/w.

*Quercus Robur* extract is an extract obtained from the plant belonging to the Phagaceae family, preferably it is a dry extract obtained from the bark of the plant.

The composition according to the invention comprising the Quercus Robur extract has anti-inflammatory, anti-edema and antioxidant properties by virtue of the presence of tannins, catechins and flavonoids such as quercetin, which promote the inhibition of pro-inflammatory cytokines such as TNF-α, IL-6, IL-8 and inducible NO (iNOS). Advantageously, the composition according to the invention comprising *Quercus Robur* extract, by virtue of a significant antioxidant and anti-inflammatory action, was particularly effective in treating lymphedema.

Advantageously, the *Quercus Robur* extract according to the present invention is titrated in tannins, preferably at 10% in tannins as measured by high performance liquid chromatography coupled with mass spectrometry.

The composition of the invention comprises an olive extract.

The olive extract according to the present invention is preferably derived from olive oil and/or olive leaves.

Advantageously, the olive extract according to the present invention is titrated in hydroxytyrosol, more preferably at 10% hydroxytyrosol measured by HPLC chromatography.

Preferably, the olive extract is present in the composition of the invention in the range from about 6% to about 24% w/w with respect to the total weight of the composition, more preferably about 12% w/w.

The composition of the invention comprising the olive extract, preferably titrated with hydroxytyrosol (HT), carries out an anti-inflammatory action and efficiently reduces the synthesis of LTB4, by inhibiting the activity of the enzyme 5-lipoxygenase, thus being effective for treating lymphedema.

Ketoprofen, a non-steroidal anti-inflammatory drug (NSAID), carries out the same mechanism of action as the composition according to the invention comprising hydroxytyrosol, inhibiting the activity of 5-lipoxygenase and regulating the synthesis of LTB4. Advantageously, the composition according to the invention comprising the olive extract titrated in hydroxytyrosol was effective for alleviating inflammation and inducing pro-lymphangiogenesis factors, thus reversing edema and restoring lymphatic function. Unlike NSAIDs, such as ketopofen, the composition according to the invention advantageously has no gastrolesive effects.

The composition of the invention can be used for the formulation of a nutraceutical product, being it a supplement or a food, by using suitable excipients.

The invention preferably relates to a supplement comprising the composition of the invention and physiologically acceptable excipients.

The composition of the invention, i.e., the nutraceutical product, the food or the supplement comprising the same, has demonstrated an excellent activity in reducing inflammation markers, in regulating vascular permeability, in the ability to reabsorb extravasation, in the maintenance of lymph node function, in the ability to restore cellular oncotic pressure and in the stimulation of lymphangiogenesis.

Therefore, in another aspect, the invention relates to the composition or nutraceutical (or supplement or food) of the invention for use in treating lymphedema.

According to a further aspect, the invention relates to the composition or nutraceutical product (or supplement or food) of the invention for use in treating edema.

In a further aspect, the invention relates to the use of the nutraceutical product in improving symptoms associated with edema or lymphedema, in which said improvement consists of reducing inflammation markers, regulating vascular permeability, the ability to reabsorb extravasation, the maintenance of lymph node function, the ability to restore cellular oncotic pressure and the stimulation of lymphangiogenesis.

The composition, i.e., the nutraceutical product, food or supplement comprising the same for use in treating edema and/or lymphedema can preferably be administered by oral administration.

The compositions can thus be in liquid, solid or semi-solid form and can be in the form of tablets, beads, drops, sticks, syrups, sachets.

To this end, the composition of the invention comprises excipients adapted to provide the final formulation in the form of a preparation unit dose.

The compositions of the invention are orally administrable in the form of tablets, capsules, granules, effervescent granules, syrups or the like.

They can also be dissolved in water to form inhalable solutions.

The formulations in solid form can comprise one or more additives such as starches, sugars, cellulose, diluents, granulating agents, lubricants, binders, disintegrating agents, stabilizers, emulsifiers, texturizers, plasticizers, wetting agents, thickeners, coatings. All of these excipients allow the supplement of the invention to be obtained in the desired pharmaceutical formulation.

The capsules can be coated with a gastro-resistant film, or they can be soft gel capsules, or, preferably, they can be in solid tablets.

Flavoring agents, preservatives, dyes, or the like can also be present.

Preferably the composition of the invention is administered orally, more preferably in tablet form.

The daily dose of the finished product is preferably two tablets comprising the composition of the invention per day.

The formulation of the invention comprising the composition of the invention comprises from 100 to 400 mg, preferably about 200 mg of bromelain with respect to a preparation unit dose.

The formulation of the invention comprising the composition of the invention comprises *Morinda Citrifolia* extract in the range from 25 to 100 mg, preferably about 50 mg with respect to a preparation unit dose.

The formulation of the invention comprising the composition of the invention, comprises *Meliloto Officinalis* extract in the range from 37.5 to 150 mg, preferably about 75 mg with respect to a preparation unit dose.

The formulation of the invention comprising the composition of the invention comprises *Quercus Robur* extract in the range from 25 to 100 mg, preferably about 50 mg with respect to a preparation unit dose.

The formulation of the invention comprising the composition of the invention, comprises olive extract in the range from 25 to 100 mg, preferably about 50 mg with respect to a preparation unit dose.

In a preferred and advantageous form of administration, the formulation is a tablet, comprising

| | |
|---|---|
| - 50 mg | *Morinda Citrifolia* extract titrated at 1% in rubiadin |
| - 75 mg | *Meliloto Officinalis* extract titrated at 40% in coumarin |
| - 50 mg | olive leaf extract titrated at 10% in hydroxytyrosol |
| - 50 mg | *Quercus Robur* extract titrated at 10% in tannins |
| - 200 mg | microencapsulated Bromelain 5000 GDU/g |

The active ingredients contained in the compositions object of the present invention can be combined or mixed as known active ingredients and/or excipients according to pharmaceutical and food or nutritional industry techniques and prepared by methods known in the pharmaceutical or food industry.

The compositions of the invention are also used as adjuvants in the therapies for treating edema and/or lymphedema.

Therefore, in a further advantageous aspect of the invention, the composition of the invention or the nutraceutical product according to the invention can be combined with at least one known drug, preferably in treating edema and/or lymphedema. Therefore, in another aspect, the invention relates to a combination comprising
- the composition according to the invention or the nutraceutical according to the invention and
- a drug
for simultaneous or separate use in treating edema and/or lymphedema

In fact, the composition of the invention, i.e., the nutraceutical product, supplement or food, can be advantageously employed as a co-therapeutic agent in a combination therapy with a known drug.

Advantageously, said known drug is selected from the group consisting of a non-steroidal anti-inflammatory drug (NSAID), preferably selected from the group consisting of acetylsalicylic acid, ibuprofen, naproxen, ketoprofen and diclofenac, a diuretic drug, preferably furosemide, and an anti-edema drug, preferably selected from the group consisting of heparan sulfate, troxerutin and diosmin, more preferably it is a NSAID, even more preferably it is ketoprofen.

The composition of the present invention, i.e., the nutraceutical product, the supplement or the food of the invention, is thus a valid therapeutic tool for managing the symptoms of edema and/or lymphedema.

The synergism generated by the above combination has shown the effectiveness thereof in enhancing the effects of the components thereof, which was greater than the sum of the known effects of the individual active ingredients.

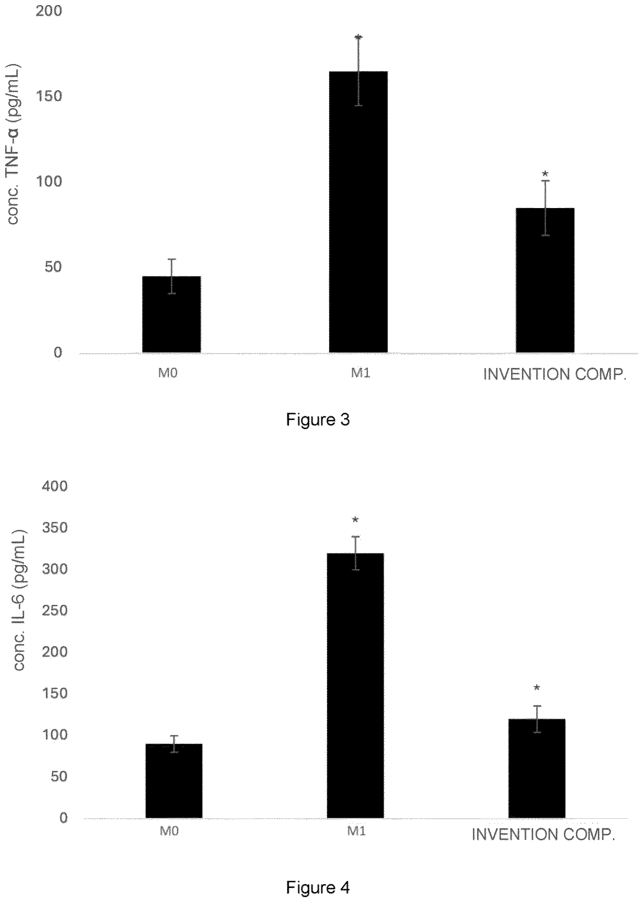

As will be clear from the subsequent experimental section, the composition of the invention has demonstrated a synergistic anti-inflammatory effect, allowing a significant inhibition of pro-inflammatory M1 macrophages after *in vitro* stimulation with acetic acid. Furthermore, the composition demonstrated a stimulatory effect for producing anti-inflammatory M2 macrophages with a consequent increase in albumin phagocytosis in the lymphatic pathways and reduced lymphatic extravasation. Advantageously, the composition of the invention has revealed being able of significantly contributing to the reduction of the spread of phlogosis mediators such as IL-1β, IL-6 and TNF-α, as can be seen from Figures 3, 4 and 5.

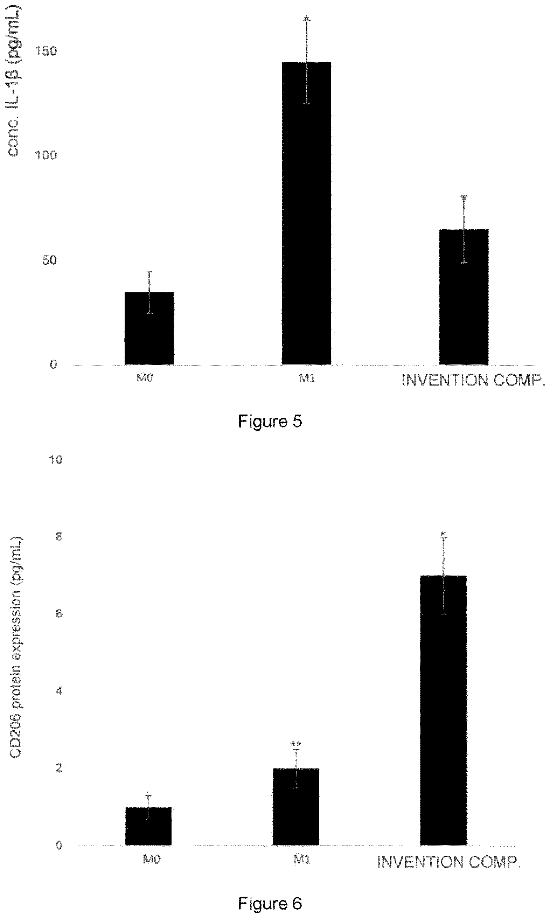

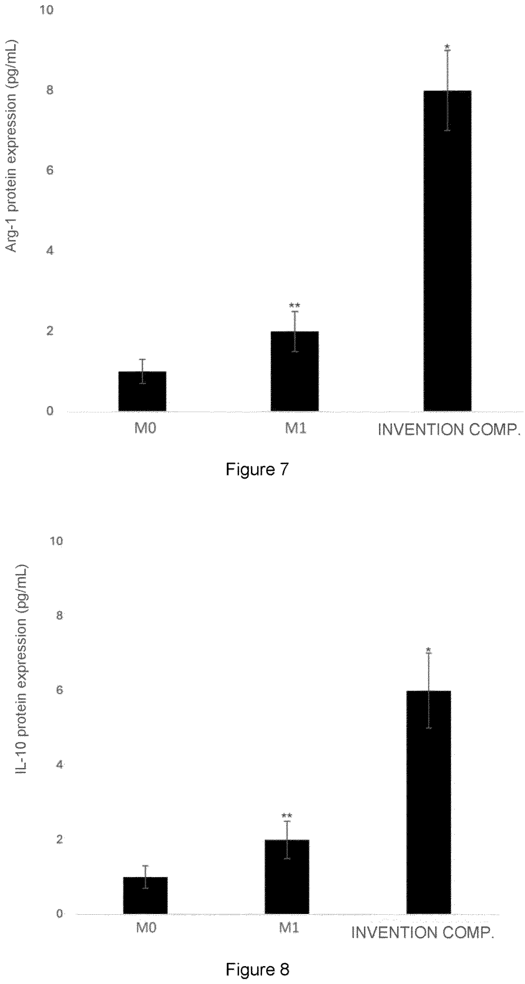

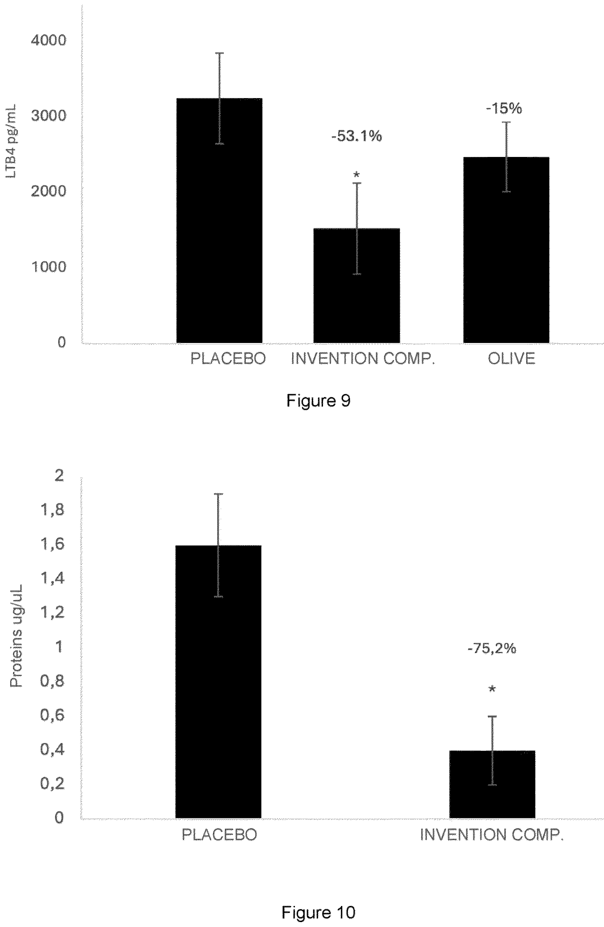

Furthermore, the composition of the invention stimulated the diffusion of anti-inflammatory molecules such as 10 IL-10 interleukins and M2 macrophages. In fact, it can be seen from Figures 6-8 that the group of cells of Example 3 treated with the composition of the invention showed an increase in the levels of M2 macrophages with respect to the control group M0 and the group M1 not treated with the composition of the invention. The increase in M2 macrophage levels is clearly visible from the increase in CD206 concentration in Figure 6, the increase in Arg-1 in Figure 7, and the increase in the anti-inflammatory interleukin IL-10 in Figure 8, all related to M2 macrophages. Surprisingly, the composition of the invention has revealed being capable of selectively reducing blood levels of leukotriene LTB4, an extremely pro-inflammatory molecule involved in all phlogosis mechanisms. As can be seen from Figure 9, treatment with the composition reduced the concentration of LTB4 by more than 53%, contributing to the reduction of the recall of other macrophages and neutrophils at the site of phlogosis and the vicious cycle leading to the synthesis of other M1 macrophages.

Lastly, as is clear from Figure 10, the composition was advantageously capable of significantly reducing the blood concentration of extravasated proteins in the treated subjects. This phenomenon is of fundamental importance in reducing edema and lymphedema, since the lower concentration of blood flow solutes represents the first stage of extravasation resorption.

### EXPERIMENTAL SECTION

### Example 1: preparation of the composition of the invention

For the preparation of the composition of the invention and thus of the final product, for example a supplement, the following were used:

| | |
|---|---|
| - 50 mg | *Morinda Citrifolia* extract titrated at 1% in rubiadin |
| - 75 mg | *Meliloto Officinalis* extract titrated at 40% in coumarin |
| - 50 mg | olive leaf extract titrated at 10% in hydroxytyrosol |
| - 50 mg | *Quercus Robur* extract titrated at 10% in tannins |
| - 200 mg | microencapsulated Bromelain 5000 GDU/g |

All quantities were expressed with respect to unit dose preparations.

The compositions of the invention could be formed by combining the above ingredients in the quantities indicated.

### Example 2: Evaluation of the composition of the invention.

### 2.1 Tested composition

The composition of the invention was tested by an in vitro study for the evaluation of a nutraceutical formulation in treating edema and/or lymphedema, and related symptoms.

The tested composition of the invention comprised:
- *Morinda Citrifolia* extract 10.4 mg,
- *Quercus Robur* extract 10.4 mg,
- *Meliloto Officinalis* extract 15.6 mg,
- microencapsulated Bromelain 32.2 mg, and
- olive extract 10.4 mg.

The study was carried out at the Department of Pharmacy, University of Naples "Federico II".

Evaluations were carried out to demonstrate the ability of the product to be used in treating edema and/or lymphedema.

The study was aimed at evaluating the ability of the nutraceutical product of the invention to reduce inflammation markers, regulate vascular permeability, reabsorb extravasation, maintain lymph node function, restore cellular oncotic pressure and stimulate lymphangiogenesis.

### 2.2. Experimental model

Six healthy male Wistar, Albino Swiss rats weighing 150-250 g (average 200 g) were used.

All animals (n=6) were kept in approved plastic cages, with wire mesh lids and bottoms, at a temperature of 20 ± 2°C and were exposed to a 12-hour dark-light cycle. Food and water were provided *ad libitum* for the entire duration of the study.

### 2.3. Induced edema test

Carrageenan (lambda form, FMC Marine Colloids Division, NJ, or type IV, Sigma-Aldrich, Poole, UK) was employed as a 1% w/v solution in 0.9% saline. In each rat, an aliquot of 100 µL was injected subcutaneously into the plantar region of the left hind leg. The injections were made under slightly anesthetized conditions (ketamine hydrochloride, 20 mg/kg, intraperitoneal) using a 5/8 gauge 25 needle inserted into the hairless skin of the pad region on the underside of the leg. All the rats (n=6) used for *in vivo* testing were healthy. Three of them were treated only with a stimulating agent for inflammation and extravasation (acetic acid), and the remaining three were instead treated with the same stimulating agent and the composition of the invention.

### 2.4. Treatment

Treatment (n=6) with the composition of the invention indicated in point 2.1 included the oral administration of an aliquot thereof by gavage technique. The treatment consisted of a single dose of the composition of the invention 1 hour before the injection of carrageenan.

### 2.5. Measurement of leg swelling

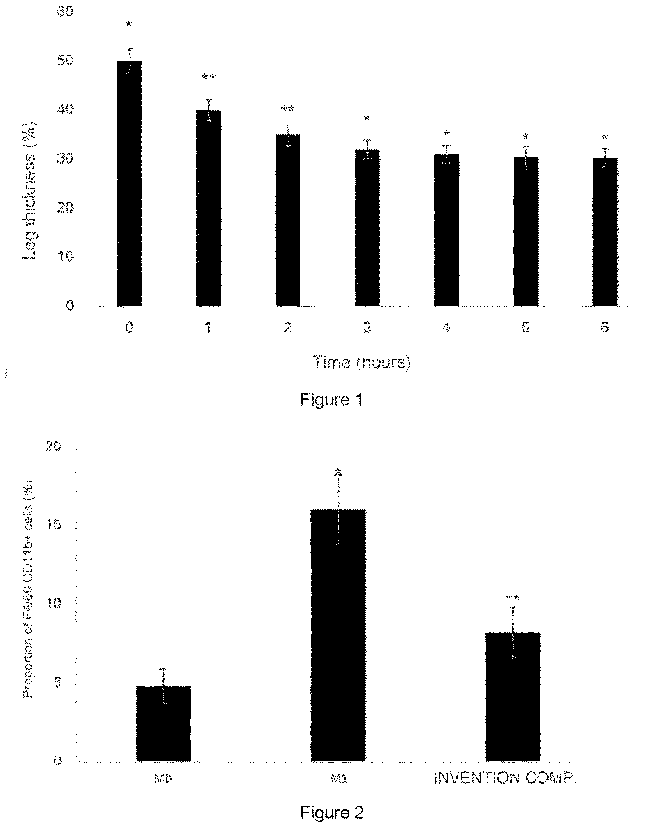

Edema was assessed by measuring the thickness of the leg in the dorsoplantar region at the metatarsal level by calipers. The measurement point was pre-marked on the upper part of the foot with an indelible pen for reference. Edema was assessed by measurement of the injected paw initially and 1, 2, 3, 4, 5 and 6 hours after carrageenan injection. A digital caliper was used, multiple measurements were taken at a given time and the results entered into a computer. A leg thickness index is calculated as an average difference of the leg thickness (Δ leg thickness/contralateral leg thickness) (Figure 1).

### 2.6. Determination of the effect on vessel permeability

The same rats (n=3) treated with the composition of the invention indicated above in Example 2.1., were used for injections of 0.1 mL of 4% Evans blue (Sigma, St. Louis, Missouri, USA) in saline into the tails, 30 minutes after treatment. A 0.4 mL aliquot of 0.5% (v/v) CH₃COOH (acetic acid) was injected i.p. (intraperitoneal) 10 minutes after intravenous injection of the dye solution. After 20 minutes, the mice were sacrificed and the bowels were irrigated with distilled water by means of 10 mL graduated flasks, through glass wool. The absorption of the final solution was measured at 590 nm (Beckmann Dual Spectrometer), after each flask was brought to a volume of 10 mL with distilled water, and 0.1 mL of a 0.1 N NaOH solution was added. A mixture of distilled water and sodium carboxymethylcellulose (0.5%) was used for the control group (n=3) consisting of the 3 rats in which phlogosis and extravasation were induced by injection of acetic acid. The results are shown in Table 1.

**Table 1. Inhibition effect of the sample on the increase of capillary permeability induced by acetic acid.**

| **Test** | **Evans Blue (ug/mL) ± SD** | **Inhibition (%)** |
|---|---|---|
| Control (acetic acid) (n=3) | 17.2 ± 2.1 | - |
| Composition of the invention + acetic acid (n=3) | 8.8 ± 1.7 | 48.8* |
| *P<0.01 | | |

The results shown in Table 1 allow the reduction of extravasation caused by induced inflammation to be established in a clear, displayable manner. The injection of acetic acid into the tissues of the rats of the control group (n=3) generates acute inflammation and leakage from the blood and lymphatic flow of proteins and immune cells which call up water in the extra-vasal tissues (edema). By staining such liquids with 4% Evans blue in the injection area (tail) and monitoring the presence of staining in the bowels, the change in tissue permeability was evaluated. The data showed a reduction of extravasation in the subjects treated with acetic acid (stimulating agent of phlogosis) and the composition of the invention of about 50%.

### Example 3:

### Determination of the effect on M macrophage polarization

### Cell line and M1 polarization

RAW264.7 macrophage culture (Wanleibio, Shenyang, China) was grown in DMEM containing 10% fetal bovine serum in a 37°C incubator with 5% CO₂ (HF-90, Lishen Co., Shanghai, China). The experimental groups were as follows: (1) M0 group, RAW264.7 cells cultured under normal conditions; (2) M1 group, the cells were treated with 500 ng/mL LPS (L8880, Solarbio, China) and 20 ng/mL IFN-γ (Z02916, Ginrys, China) for 12 hours and the morphology of each group was observed under a microscope (IX53, Olympus, Japan), after which, the groups of M0 and M1 cells in good condition were selected for cell treatment; (3) group of the invention in which the group of M1 cells were treated with a composition of the invention comprising:
- Morinda Citrifolia extract 50.8 mg,
- Quercus Robur extract 50.8 mg,
- Meliloto Officinalis extract 75.0 mg,
- microencapusulated bromelain 153.8 mg,
- olive extract 50.8 mg.

The cells of each group were cultured for 24 hours and then the analysis was carried out. The results are shown in Figure 2.

As can be seen from Figure 2, the treatment group with the composition of the invention showed a significant reduction in the synthesis of pro-inflammatory M1 macrophages. The reduced presence of such macrophages was indicative of the reduction in inflammation and edema, as M1 caused the increased synthesis of phlogosis mediators.

### Example 4:

### 4.1. Macrophage detection by flow cytometry

For the macrophage detection test by flow cytometry, the RAW264.7 macrophage culture of Example 3 was used. The purpose of the test was to indicate and quantify by means of a numeric value the reduction in the presence of M1 macrophages (pro-inflammatory) and at the same time to search in the treated tissues for the presence of the mediators of phlogosis which derive directly from the presence of M1 (i.e. IL-1 B, IL-6 and TNF-α). To express the tissue presence of macrophages and various immune molecules by a numerical value, specific binding antibodies (F4/80 and CD11b) were used. This process makes it possible to accurately identify the tissue presence of a molecule by means of the reference antibody. In fact, the antibody-target protein binding is very specific and allows it to be easily isolated.

The RAW264.7 macrophage culture was centrifuged (350 × g, 5 min) and cells from each group were harvested and washed twice with PBS. The dye buffer was added to resuspend the cells, the quantity of F4/80 antibody (123109, Biolegend, USA) was 0.5 µg, and the quantity of CD11b antibody (101211, Biolegend, USA) was 0.25 µg; the cells were incubated under ice for 20 minutes without light. The cells were centrifuged (350 × g, 5 min) and the supernatant was discarded. The dye buffer (500 µL) was added to each tube and the cells were resuspended slowly and completely. The cells were detected by flow cytometry (NovoCyte, Eisenbio, USA).

### 4.2. Western blotting

Cells in each group of Example 3 were lysed with RIPA lysis buffer for total protein extraction.

The purpose of the test was to demonstrate the increase in the presence of (anti-inflammatory) M2 macrophages and at the same time to search the treated tissues for the presence of the M2-binding surface receptors, CD206 and Arg-1, and to measure the expression of IL-10, also related to M2 macrophages. The protein concentration of each sample was measured using a quantitative BCA protein kit. A 10 µL aliquot of the protein was subjected to 10% SDS-PAGE and separated by 150 V constant pressure electrophoresis for 1 h. The proteins were then transferred to a PVDF membrane, which was blocked with 5% skim milk powder for 2 h and stirred at 25°C for 1 h. The following primary antibodies were added against: iNOS (1:400 WL0992a), CD68 (1:1,000 WL04839) IL-6 (1:500, WL02841) TLR4 (1:1,000, WL00196), TNF-α (1:1,000, WL01581), IL-10 (1:1,000, WL03088), Arg-1 (1:1,000, WL02825), p-JAK2 (1:300, WL02997), JAK2 (1:500, WL02188), p-STAT3 (1:500 WLP2412), STAT3 (1:1.000, WL03207), CD36 (1:500, WL02390) and PPARγ (1:1.000, WL01800), all from Wanleibio, CD206 (1:500, A11192), ABCA1 (1:1.000, A7228) and LXRα (1:1.000, ABclonal), all from ABclonal (ABclonal Technology Co., Ltd. Wuhan, China), and ABCG1 (1:1000, ab52617, Abcam). The antibodies were incubated overnight at 4°C. The membrane was washed with TBST three times, incubated with anti-rabbit IgG-HRP secondary antibody (1:5000), except for p-JAK2 antibody (sheep anti-mouse IgG-HRP at 1 :5000) at 37°C for 45 min, then rinsed with TBST three times. The gray values of the target protein and the bands of β-actin (internal reference protein) were compared using an E-Gel Imager (Gel-Pro-Analyzer, WD- 9413B, Beijing Liuyi Biotechnology Co., Beijing, China).

The results of the tests in 4.1. and 4.2 are shown in Figures 3-8.

Thus, as can be seen from the experimental tests and the figures, the composition of the invention showed a synergistic anti-inflammatory effect allowing a significant inhibition of pro-inflammatory M1 macrophages after *in vitro* stimulation with acetic acid. Furthermore, the composition demonstrated a stimulatory effect for producing anti-inflammatory M2 macrophages with a consequent increase in albumin phagocytosis in the lymphatic pathways and reduced lymphatic extravasation. Advantageously, the composition of the invention has revealed being able of significantly contributing to the reduction of the spread of phlogosis mediators such as IL-1β, IL-6 and TNF-α, as can be seen from Figures 3, 4 and 5.

Furthermore, the composition of the invention stimulated the diffusion of anti-inflammatory molecules such as 10 IL-10 interleukins and M2 macrophages.

In particular, it can be seen from Figures 6-8 that the group treated with the composition of the invention showed an increase in the levels of M2 macrophages with respect to the control group M0 and the group M1 not treated with the composition of the invention. Specifically, the increase in M2 macrophage levels is noted by the increase in CD206 receptor concentration in Figure 6, the increase in Arg-1 receptor in Figure 7, and the increase in IL-10 anti-inflammatory interleukin in Figure 8.

### Example 5: Experimental model

Six male Wistar, Albino Swiss rats weighing 150-250 g (average 200 g) were used. The healthy animals (n=6) were kept in approved plastic cages, with wire mesh lids and bottoms, at a temperature of 20 ± 2°C and were exposed to a 12-hour dark-light cycle. Food and water were provided *ad libitum* for the entire duration of the study.

### 5.1. Measurement of plasma levels of LTB₄

The efficacy of the composition in reducing leukotriene LTB4 was evaluated. The result was compared with the result of a treatment carried out with olive extract alone, which is known to be particularly effective in reducing leukotriene LTB4.

The following treatment groups were used:
- group 1: treatment with the composition of the invention (n=3);
- group 2: treatment with olive extract only (n=1);
- group 3: placebo treatment (n=2).

In particular, treatment group 1 (n=3) included the oral administration by gavage technique of a composition of the invention comprising:
- *Morinda Citrifolia* extract 10.4 mg,
- *Quercus Robur* extract 10.4 mg,
- *Meliloto Officinalis* extract 15.6 mg,
- microencapsulated Bromelain 32.2 mg,
- olive extract 10.4 mg

Group 2 (n=1) O., included the oral administration by gavage technique of an olive extract 10.4 mg.

The treatment consisted of a single dose of sample 1 hour before the injection of acetic acid.

The placebo group (n=2) received a mixture of distilled water and sodium carboxymethylcellulose (0.5%). A 0.4 mL aliquot of 0.5% (v/v) AcOH was injected i.p. After 20 minutes, an aliquot of blood was taken from the tail vein and centrifuged. The plasma was processed according to ELISA method for the measurement of LTB₄ levels.

The results are shown in Figure 9. The experiment demonstrated the reduction of the blood concentration of leukotriene LTB4, a molecule responsible for inflammation and tissue extravasation. The significant reduction thereof (over 53%), as seen in Figure 9, translates into the reduction of the recall of other M1 macrophages and the reduction of the synthesis of other inflammatory mediators which cause extravasation from the blood and lymphatic pathways.

### 5.2. Measurement of plasma protein levels

To treatment group 1 (n=3) was administered orally, by gavage technique, the composition of the invention reported in point 5.1 and a phlogosis stimulating agent (acetic acid). The treatment consisted of a single dose of the composition, 1 hour before the acetic acid injection. The placebo group (n=3) received a mixture of distilled water and sodium carboxymethylcellulose (0.5%) and acetic acid. A 0.4 mL aliquot of 0.5% (v/v) AcOH was injected i.p. After 20 minutes, an aliquot of blood was taken from the tail vein and centrifuged. The plasma protein concentration was measured by colorimetric dosing (Bio-Rad DC Protein Assay), based on the reaction of the proteins with an alkaline solution of copper tartrate and Folin reagent. The fluid supernatant obtained by centrifugation (400 g, 8 min, 4°C) was added to a 96-well ELISA plate (NUNC^{™} products, Denmark). Absorbance was read at 700 nm. To generate a reference, the serum albumin standard was used. The results are shown in Figure 10. The experiment evaluated the protein concentration in the vessel bed of the treated subjects after phlogosis stimulation with acetic acid. Under normal conditions, the concentration of proteins in the blood and extra-vasal tissues is in a state of equilibrium. With the injection of acetic acid in the tail of the subjects, an acute inflammation is established which calls up immune molecules (mainly of protein nature) from the other districts. These molecules significantly increase the protein concentration in the blood, followed by increased capillarity of the vessels. Subsequently, these protein components pass from the blood vessel to the extra-vasal tissues, modifying the pre-existing concentration balance (calling up liquids in the tissues - edema - lymphedema). The experiment demonstrated a reduction in the concentration of proteins responsible for extravasation of more than 75% in the treated subjects. This phenomenon translates into a significant reduction in the spread of edema and lymphedema and above all in the ability to reabsorb the liquids already extravasated, in the event of acute edema, as can be seen from Figure 10.

Thus, as can be seen from the experimental tests and the figures, the composition of the invention showed a synergistic anti-inflammatory effect allowing a significant inhibition of pro-inflammatory M1 macrophages after *in vitro* stimulation with acetic acid. Furthermore, the composition demonstrated a stimulatory effect for producing anti-inflammatory M2 macrophages with a consequent increase in albumin phagocytosis in the lymphatic pathways and reduced lymphatic extravasation. Surprisingly, the composition of the invention has revealed being capable of selectively reducing blood levels of leukotriene LTB4, an extremely pro-inflammatory molecule involved in all phlogosis mechanisms. As can be seen from Figure 9, treatment with the composition reduced the concentration of LTB4 by more than 53%, contributing to the reduction of the recall of other macrophages and neutrophils at the site of phlogosis and the vicious cycle leading to the synthesis of other M1 macrophages.

Lastly, as is clear from Figure 10, the composition was advantageously capable of significantly reducing the blood concentration of extravasated proteins in the treated subjects. This phenomenon is of fundamental importance in reducing edema and lymphedema, since the lower concentration of blood flow solutes represents the first stage of extravasation resorption.

## Claims

1. A composition comprising a *Morinda Citrifolia* extract, a *Meliloto Officinalis* extract, a *Quercus Robur* extract, an olive extract and bromelain.

2. A composition according to claim 1, wherein the bromelain is microencapsulated, preferably said bromelain having a proteolytic enzyme activity equal to about 5000 GDU/g.

3. A composition according to claim 1 or 2, wherein the bromelain is in a quantity in the range from about 24% to about 94%, preferably from about 40% to about 59%, more preferably about 47% w/w with respect to the total weight of the composition.

4. A composition according to any one of claims 1 to 3, wherein the *Morinda Citrifolia* extract is titrated in rubiadin, preferably at 1% rubiadin.

5. A composition according to any one of claims 1 to 4, wherein the *Morinda Citrifolia* extract is in a quantity in the range from about 6% to about 24% w/w, preferably about 12% w/w.

6. A composition according to any one of claims 1 to 5, wherein the *Meliloto Officinalis* extract is titrated in coumarin, preferably at 40% in coumarin.

7. A composition according to any one of claims 1 to 6, wherein the *Meliloto Officinalis* extract is in a quantity in the range from about 9% to about 35% w/w with respect to the total weight of the composition, preferably about 18% w/w.

8. A composition according to any one of claims 1 to 4, wherein the *Quercus Robur* extract is titrated in tannins, preferably at 10% in tannins.

9. A composition according to any one of claims 1 to 8, wherein the *Quercus Robur* extract is in a quantity in the range from about 6% to about 24% w/w with respect to the total weight of the composition, preferably about 12% w/w.

10. A composition according to any one of claims 1 to 5, wherein the olive extract is titrated in hydroxytyrosol, preferably at 10% in hydroxytyrosol.

11. A composition according to any one of claims 1 to 10, wherein the olive extract is in a quantity in the range from about 6% to about 24% w/w with respect to the total weight of the composition, preferably about 12% w/w.

12. A nutraceutical product comprising the composition according to any one of claims 1 to 11, and physiologically acceptable excipients.

13. A nutraceutical product of claim 12 which is a supplement, preferably in capsule form.

14. A composition according to any one of claims 1 to 11, or a nutraceutical product according to claim 12 or 13, for use in treating edema and/or lymphedema.

15. A combination comprising
- the composition according to any one of claims 1 to 11, or the nutraceutical product according to claim 12 or 13, and
- a drug
for simultaneous or separate use in treating edema and/or lymphedema.
